(19) Europäisches Patentamt
European Patent Office
Office européen des brevets

(11) Publication number: **0 149 347**
**B1**

(12) **EUROPEAN PATENT SPECIFICATION**

(45) Date of publication of patent specification: **09.03.88**

(51) Int. Cl.⁴: **C 07 C 135/02,**
C 07 D 295/22, B 01 F 17/00

(21) Application number: **84308871.7**

(22) Date of filing: **18.12.84**

(54) **Pentahydroperfluoroalkylamine n-oxides.**

(30) Priority: **29.12.83 US 566800**

(43) Date of publication of application:
**24.07.85 Bulletin 85/30**

(45) Publication of the grant of the patent:
**09.03.88 Bulletin 88/10**

(84) Designated Contracting States:
**AT BE CH DE FR GB IT LI LU NL SE**

(56) References cited:
**AT-B- 294 111**
**DE-B-1 518 087**
**GB-A-1 302 612**
**US-A-4 165 338**

(73) Proprietor: **E.I. DU PONT DE NEMOURS AND COMPANY**
**1007 Market Street**
**Wilmington Delaware 19898 (US)**

(72) Inventor: **Clarke, Ray Allen**
**3211 Kevington Avenue**
**Eugene, Oregon 97405 (US)**
Inventor: **Cords, Donald Philip**
**207 Hanover Place**
**Newark, Delaware 19711 (US)**

(74) Representative: **Woodcraft, David Charles et al**
**BROOKES & MARTIN High Holborn House 52/54**
**High Holborn**
**London, WC1V 6SE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background of the invention

This invention relates to 1,1,2,3,3-pentahydroperfluoroalkylamine N-oxides having a 2-hydroxy or acyloxy substituent and processes for making and using the same as surfactants and foaming agents.

U.S. Patent No. 4,283,533 (Richter), discloses amphoteric N-type betaines of 2-hydroxy-1,1,2,3,3-pentahydroperfluoroalkylamines and their use as surfactants. U.S. Patent No. 4,165,338 (Katsushima et al), discloses various hydroxy compounds including ones having the formula:

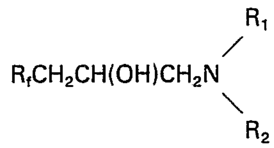

wherein $R_1$ and $R_2$ are each hydrogen or a hydrocarbon group, including

$CF_3(CF_2)_6CH_2CH(OH)CH_2N(C_2H_5)_2$ and $CF_3(CF_2)_6CH_2CH(OH)CH_2N(CH_3)_2$,

among others. Katsushima et al, disclose acid addition salts of their compounds.

Minnesota Mining and Manufacturing Company, in British Patent No. 1,302,612, discloses fluorinated amine N-oxide surfactants, some of which can be represented by the formula:

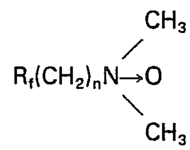

wherein n is 1—3. Amongst others, data are provided as to the foaming characteristics of the amine N-oxides.

The compounds of the present invention overcome a number of disadvantages associated with the compounds of Richter, and Katsushima et al. In order to prepare their betaines, Richter react a tertiary amine, or mixture of tertiary amines, with an alkali metal salt of a monochloro or monobromo alkanoic acid. That type of reaction is much more difficult to perform effectively than any of the oxidation techniques described herein. Katsushima et al. report their compounds are oleophobic, not surface active.

The compounds of the present invention are more effective in reducing surface tension of aqueous solutions at low concentrations than those of the Minnesota Mining and Manufacturing Company British patent. Moreover, the amine N-oxides of the present invention are markedly superior aqueous foaming agents when compared with the amine oxides disclosed by Minnesota Mining and Manufacturing Company in the British patent. Moreover, the amine oxides of the Minnesota Mining and Manufacturing Company British patent are much more difficult to prepare than those of the present invention.

Detailed description of the invention

The amine N-oxides of this invention can be represented by the formula:

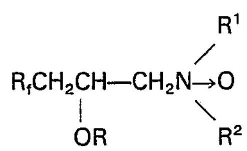

wherein

$R_f$ is $C_3$—$C_{20}$ perfluoroalkyl;

R is H or alkanoyl containing 1—4 carbons;

$R^1$ and $R^2$ are the same or different $C_1$—$C_6$ alkyl or $C_1$—$C_6$ hydroxyalkyl, or together with the N atom to which they are bonded form a piperidine, morpholine or N-alkyl($C_1$—$C_4$)piperazine ring; or

$R^1$ is $C_1$—$C_6$ alkyl or $C_1$—$C_6$ hydroxyalkyl and

$R^2$ is 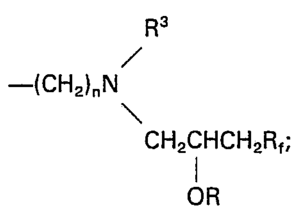

$R^3$ is $C_1$—$C_6$ alkyl or $C_1$—$C_6$ hydroxyalkyl; and

n is an integer from 1—8; and mixtures thereof.

The amine N-oxides of this invention are highly effective in reducing the surface tension of water and of aqueous solutions of inorganic electrolytes. Because they are essentially nonionic, they can be used in environments in which ionic surfactants may fail, e.g., highly acidic oil well applications. The amine N-oxides of this invention are useful also as aqueous foaming agents. They may therefore find use in foam fracturing of petroleum-bearing formations and in fire extinguisher compositions, particularly those used for extinguishing petroleum fires. Moreover, compositions containing them exhibit improved insecticidal properties, e.g., against German cockroaches.

This invention contemplates single compounds represented by the foregoing formula as well as mixtures thereof; preferably the latter are prepared and used, because the starting materials which provide the perfluoroalkyl portion of the molecule are most commonly available commercially as mixtures. The usual commercial mixture will contain small amounts (less than 5% by weight) on both ends of the foregoing carbon chain length range, with 80—90% of the chains containing 6—12 carbons, preferably 6—10 carbons, with an average in the mixture between 6 and 8 carbons.

The amine N-oxides of this invention can be prepared by reacting the appropriate 2-hydroxy or 2-acyloxy tertiary amine (or mixture of 2-hydroxyamines and/or 2-acyloxyamines) with an oxidizing agent. For example, one can react the amine or mixture of amines with hydrogen peroxide or peracetic acid in an alcohol or aqueous alcoholic solvent, such as methanol, ethanol, 1-propanol, 2-propanol, 1-butanol, 2-methyl-2-propanol and mixtures thereof. Preferably, the oxidizing agent is peracetic acid, and the oxidation reaction is run in 2-propanol.

The acyloxy tertiary amines can be prepared by reacting the corresponding 2-hydroxy tertiary amine with any appropriate acylating agent. While an acid anhydride is preferred, other acylating agents, such as acids and acid halides, may be used. The acylating reaction can be run neat or in a suitable nonpolar solvent or aproptic polar solvent, e.g., ketones such as methyl ethyl ketone; aromatic hydrocarbons such as toluene and benzene; diethyl ether; pyridine; alkanoic acids such as acetic, propanoic, butanoic or pentanoic acid; and the like. In a preferred embodiment, the acylation reaction is run in the absence of any reaction medium other than the amine and the acylating agent. The reaction product, in some instances, is reacted with the oxidizing agent without recovery of the acylation reaction product; in others, the reaction product is purified before it is oxidized, preferably the former.

Some of the 2-hydroxyamines, from which some of the 2-hydroxyamine N-oxides and 2-acyloxyamine N-oxides are prepared, are disclosed by Richter and Katsushima et al., supra. Those 2-hydroxyamines, as well as the others which serve as starting materials for the amine N-oxides of this invention, can be prepared by the reaction:

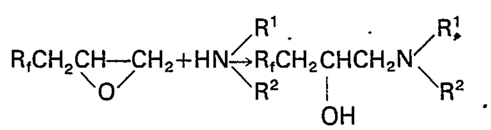

The epoxide starting material for the foregoing reaction can be prepared by reacting an alkali metal hydroxide with 3-perfluoroalkylpropylene iodohydrin, having the formula:

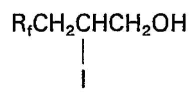

Some of the iodohydrins are known compounds; Richter and Katasushima et al., supra, disclose some of them and their preparation. Moreover, they can be prepared, as is more particularly described hereinbelow by reacting the appropriate perfluoroalkyl iodide with triallyl borate in the presence of a free radical initiator, following which the resulting borate is hydrolyzed to the iodohydrin. The preparation of triallyl borate is described by Richter, supra.

The following examples are given by way of illustration not limitation. Unless specified otherwise, all parts and percentages are by weight, and temperatures are in degrees Celsius. Surface tension data are given in Table I. The Ross-Miles Foam Test to which reference is made in the Examples is described in Oil and Soap, XVIII No. 1, 99—102 (1941). For purposes of the Examples herein, the Ross-Miles Foam Test has been modified in that deionized water is used at ambient temperature rather than distilled water at about 43°C.

I. The following preparation of 3-perfluoroalkyliodohydrins was made for Example 1, and it is typical of preparations made for Examples 2—5.

Preparation of 3-perfluoroalkyliodohydrins

A mixture of perfluoroalkyl iodides was used which had the characteristics given below. The mixture was analyzed by gas chromatography, using a Perkin-Elmer Model 3920 flame ionization gas chromatograph, a 12′×1/8″ stainless steel column packed with 20% GE SE-30 silicone on 80/100 Chromosorb W-HP and a program of heating from 50°C to 260°C at 16°C/minute. The homolog distribution was expressed as area % and the average molecular weight calculated therefrom to give the following:

| | | |
|---|---|---|
| $C_4F_9I$ | — | trace |
| $C_6F_{13}I$ | — | 51.36% |
| $C_8F_{17}I$ | — | 34.24% |
| $C_{10}F_{21}I$ | — | 9.94% |
| $C_{12}F_{25}I$ | — | 3.68% |
| $C_{14}F_{29}I$ | — | 0.72% |
| $C_{16}F_{33}I$ | — | 0.05% |
| Non-iodides | — | 0.01% |

Average molecular weight as $R_fI$=528.6.

A mixture of 644 kg 1.28 kg-mols of the above-described $R_fI$ and 304 lbs of triallyl borate (85% purity: 0.82 kg-mols) was heated to 67±3°C and Vazo® 64 azobisisobutyronitrile (1.36 kg); 0.0083 kg-mol) added, resulting in a gentle exotherm lasting approximately 2 hours. The charge was held under agitation at 67±3°C for 3 hours, after which another 1.36 kg increment of Vazo® 64 was added. The charge was agitated at 67±3°C for 3 hours, after which increments of Vazo® 64 were added as follows: 1.36 kg; 3 hours later 1.36 kg; 3 hours later 1.36 kg lbs; 3 hours later 1.36 kgs. After a total of 27 hours, the charge was sampled and the sample hydrolyzed with aqueous acetone and the hydrolyzed sample extracted into "F-113" 1,1,2-trichloro-1,2,2-trifluoroethane. Gas chromatographic analysis of the "F-113" layer showed a >99.5% conversion of $R_fI$ to 3-perfluoroalkylpropylene iodohydrins. To the charge was added 453.5 kgs of 5% sodium chloride brine; the mixture was distilled to a head temperature of 98°C (pot temperature 102°C) to remove unreacted allyl alcohol and the major portions of the toluene and unreacted $R_fI$. The two-phase still residue was separated at 83±3°C, the lower (product) layer being drummed and the upper (aqueous brine) layer being discarded, to yield 698 kgs (97.2%) of a yellow-orange oil which solidified on cooling.

Analysis (area % and mol. wt. calculated therefrom):

| | | |
|---|---|---|
| $C_4F_9CH_2CHICH_2OH$ | — | 1.20% |
| $C_6F_{13}CH_2CHICH_2OH$ | — | 48.41% |
| $C_8F_{17}CH_2CHICH_2OH$ | — | 31.98% |
| $C_{10}F_{21}CH_2CHICH_2OH$ | — | 9.61% |
| $C_{12}F_{25}CHICH_2OH$ | — | 1.54% |
| $C_{14}F_{29}CHICH_2OH$ | — | 1.18% |
| Non-iodohydrins | — | 6.08% |

Average molecular weight as $R_fCH_2CHICH_2OH$=592.1

Example 1

N,N-dimethyl(3-perfluoroalkyl-2-hydroxypropyl)amine N-oxides

Isopropyl alcohol, anhydrous (605 kgs) was warmed to 40°, and the above-described mixture of perfluoroalkylpropylene iodohydrins (463.5 kgs, 0.78 kg-mol) was added. The resultant solution was then cooled to 15±5°, and sodium chloride (189 kgs) was added. Cooling was applied to the reaction vessel as aqueous sodium hydroxide (50%, 57 kgs, 0.71 kg-mol) was added over a 30—45 minute period. The resultant mass was stirred at 5±5° for 4 hours. Dimethyl amine (40% aqueous solution, (36 kgs, 1.21 kg-mol) was added, with concomitant cooling of the reaction vessel, and the charge was stirred for an additional 4 hours at 5±5°.

While maintaining a temperature of 5±5°, aqueous sodium hydroxide (50%, 6.3 kg, 0.079 kg-mol) was added slowly. The charge was stirred for 8 hours at 5±5°, 4 hours at 25±5°, and 80±2° for 4 hours. The charge was then diluted with 1059 kgs of water.

Next, heating was applied to effect the distillation of isopropanol at a head temperature of 93±1° and a pot temperature of 101−2°. After distillation was complete, the charge was cooled to 55±5°. Agitation was stopped and the layers were allowed to separate. The lower (product) layer, 370 kgs of a dark amber oil, was drawn off. Analysis of the product by gas chromatography revealed the following composition:

| $R_fCH_2CH(OH)—CH_2N(CH_3)_2$ | Area % |
|---|---|
| $R_f$=$C_4F_9$ | 1.8 |
| $C_6F_{13}$ | 51.4 |
| $C_8F_{17}$ | 29.8 |
| $C_{10}F_{21}$ | 10.6 |
| $C_{12}F_{25}$ | 1.8 |
| $C_{14}F_{29}$ | 0.4 |
| Unknowns | 3.8 |

By amine titration the amine product had an apparent molecular weight of 537.6.

To 215 g (0.4 mol) of the mixture of N,N-dimethyl(3-perfluoroalkyl-2-hydroxypropyl)amines were added 145 g of isopropyl alcohol. Peracetic acid, 40% solution (83.6 g; 0.44 mol), was added dropwise to the isopropyl alcohol-amine solution while cooling in a cold water bath to prevent the exothermic reaction from going above 35°C. After about one-half of the peracetic acid had been added, the reaction mass tended to foam. The foam was broken by warming to 40 to 45°C, and the remaining peracetic acid was added, allowing the temperature to rise to 50°C. The pale yellow solution was heated for an additional 2 hours at 50°C. The reaction was diluted with 70 g of ethylene glycol and bottled. The calculated concentration of amine oxides in solution was 43%.

In the Ross-Miles foam test, at 0.1% concentration of the amine N-oxide product in deionized water at 25°C, foam was generated to a height of 140 mm and remained at that height for a 10-minute period.

Example 2

N,N-dimethyl(3-perfluoro-2-acetoxypropyl)amine N-oxides

To 1080 g (2.00 mols) of a mixture of N,N-dimethyl(3-perfluoroalkyl-2-hydroxypropyldimethyl)amines were added 510 g (5.00 mols) of acetic anhydride. The exothermic reaction caused the temperature to rise to 70 to 73°C. The temperature was increased to 75 to 80°C and held for 2 hours to complete the reaction. The reaction mass was cooled to about 25°C and added to 2 liters of cold water. The solution was neutralized to pH 7.0±0.1 by the addition of 30% sodium hydroxide solution (1008 g; 7.58 mols). Ice was added as needed to keep the temperature in the range of 20 to 30°C. The heavy oil product was allowed to separate and the top water layer was siphoned off. The product was washed twice with 1.5 liters of water and finally separated in a separatory funnel to obtain 1078 g of product (apparent average molecular weight by amine titration was 592).

To a solution mix of 178 g (0.3 mol) of the mixture of N,N-dimethyl(3-perfluoroalkyl-2-acetoxypropyl)amines and 89 g of isopropyl alcohol were added dropwise 62.7 g (0.33 mol) of 40% peracetic acid solution. The reaction temperature was maintained during the addition at less than 35°C by cooling in a cold water bath. The reaction temperature was thereafter increased to 50°C and held for 2 hours at that temperature. The solution was diluted with 90 g ethylene glycol and bottled. The calculated amine oxide concentration of the solution was 43.4%.

In the Ross-Miles foam test, at 0.1% concentration of the amine N-oxide product in deionized water at 25°C, foam was generated to a height of 190 mm which persisted for a 10-minute period.

Example 3

N,N-diethyl(3-perfluoroalkyl-2-acetoxypropyl)amine N-oxides

Diethylamine (14.6 g; 0.2 mol) was reacted with 3-perfluoroalkylpropylene oxide (60 g; about 0.1 mol) by heating initially for one hour at 70 to 75°C. The reaction temperature was gradually increased to 93 to 95°C and held for 6 hours. The product was then extracted with 5% brine solution (100 cc) at 60 to 70°C to remove excess diethylamine from the product. The extraction was repeated 3 more times. The final product weighed 58 g.

Analysis: For N,N-dimethyl(3-perfluoroalkyl-2-hydroxypropyl)amines with theoretical average molecular weight of 485, milliequivalents amine/g:

| | |
|---|---|
| Calculated: | 2.06 |
| Found: | 1.75 |

Thus, the apparent purity was 84.8% and the apparent average molecular weight was 571.

The above hydroxy amines (28.6 g; 0.05 mol) were acetylated with acetic anhydride (10.2 g; 0.1 mol) by heating at 85 to 90°C for one hour and 15 minutes. Without isolation of the acetoxy derivative, the reaction mass was cooled and diluted with isopropyl alcohol (12 g) and reacted with hydrogen peroxide 30% solution (7.4 g; 0.065 mol). The initial exothermic reaction to 55°C was continued at 70 to 75°C for 5 hours to complete the oxidation.

Example 4

N-butyl-N-methyl(3-perfluoroalkyl-2-acetoxypropyl)amine N-oxides

The process of Example 3 was repeated using N-methyl-N-butylamine (17.4 g; 0.2 mol). The product was extracted hot with 5% brine acidified to pH 5.7 with hydrochloric acid to remove excess N-methyl-N-butylamine.

The preparation of a mixture of acetoxy derivatives without isolation and its oxidation with hydrogen peroxide to the amine N-oxide solution was carried out by the same procedure as in Example 3.

Example 5

N-(3-perfluoroalkyl-2-acetoxypropyl)piperidine N-oxides

The reaction of 3-perfluoroalkylpropylene oxides (60 g; ca. 0.1 mol) with piperidine (11 g; 0.13 mol) was carried out in a manner similar to that for Example 3. Excess piperidine was removed by extraction with 100 cc water at about 60°C. The extraction was repeated 2 more times. The product (63.5 g) was a solid at room temperature.

Analysis: For a mixture of telomers:

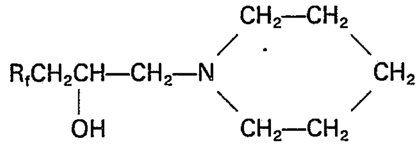

derived from a mixture of perfluoroalkyl iodides similar to that described above but with the average molecular weight of 511 the milliequivalents of amine/g:

| | |
|---|---|
| Calculated: | 1.96 |
| Found: | 1.70 |

Therefore, the purity was 87% and the apparent average molecular weight was 588.

Acetylation of the N-(3-perfluoroalkyl-2-hydroxypropyl)piperidines (60.2 g; 0.102 mol) with acetic anhydride (20.5 g; 0.201 mol) was carried out at 80 to 85°C for one hour. The reaction mass was drowned in water, neutralized with caustic soda and isolated by the general procedure of Example 2.

Analysis: Milliequivalents amine/g:

| | |
|---|---|
| Calculated: | 1.81 |
| Found: | 1.62 |

Therefore, the apparent purity was 89.6%.

N-(3-perfluoroalkyl-2-acetoxypropyl)piperidines (24.7 g; 0.04 mol) in isopropyl alcohol (20 g) were oxidized with 30% hydrogen peroxide (5.4 g; 0.048 mol) at 70 to 75°C for 3 hours. The calculated content of amine N-oxides was 50.7%.

II. The following preparation of 3-perfluoroalkylpropylene oxide was made for Example 6, and it is typical of preparations made for Examples 7—9. The iodohydrin starting material was prepared by a procedure similar to that disclosed hereinabove as well as to that disclosed by Richter.

3-Perfluoroalkylpropylene oxide

To isopropyl alcohol (544 g) was added a mixture of 3-perfluoroalkyl iodohydrins (400 g; 0.677 mol), and the mixture was heated to 45 to 50°C to dissolve the iodohydrin. The solution was then cooled in an ice bath to 7 to 10°C and 92.4 g (0.69 mol) of 30% sodium hydroxide solution were added gradually over a 4-hour period. The reaction mass was cooled to 2 to 5°C during the addition of the caustic solution and that temperature range was maintained for an additional 16 hours after the caustic soda had been added. The reaction temperature was then increased to 10 to 15°C and 8.3 g (0.06 mol) of monobasic sodium phosphate were added to neutralize excess caustic soda. The reaction mass was diluted with 800 cc water and the mixture, having a pH of 6.5, was warmed to 40 to 45°C. The heavy light brown oil product was drawn off in a separatory funnel. The product (325 g) was analyzed by gas chromatography and found to contain 12.2 area % isopropyl alcohol and had the following distribution of 3-perfluoroalkylpropylene oxides (on a sovlent-free basis):

| | Area % |
|---|---|
| 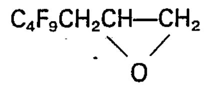 | 1.0 |
| 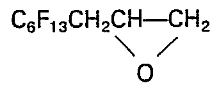 | 37.2 |
| 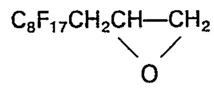 | 32.5 |
| 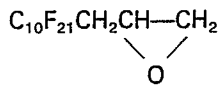 | 17.6 |
| 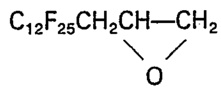 | 6.2 |
| 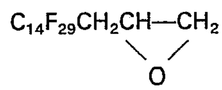 | 1.5 |
| 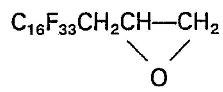 | 0.6 |
| Unknowns | 3.4 |

The calculated average apparent molecular weight of the product based upon the GC data was 470. On standing at room temperature, the product solidified partially.

Example 6

N,N-dimethyl(3-perfluoroalkyl-2-acetoxypropyl)amine N-oxides

A portion (292 g; 0.527 mol) of the crude epoxide product was reacted with diethylamine (66 g; 0.90 mol) initially at 70 to 75°C. The reaction temperature was increased gradually to 80 to 84°C over about a period of one hour and held in that temperature range for 9 hours. Water (400 cc) was added and the reaction mass subjected to distillation until the head temperature was 99°C, thereby removing excess diethylamine and isopropyl alcohol carried over from the epoxide preparation. The product, a light brown oil (283 g), was separated from the aqueous phase in a separatory funnel.

Analysis: For a mixture of telomers:

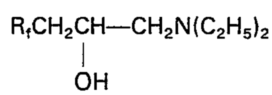

with a calculated average molecular weight of 543 the milliequivalents of amine/g:

| | |
|---|---|
| Calculated: | 1.84 |
| Found: | 1.71 |

Thus, amine product had an apparent purity of 92.4% and an apparent average molecular weight of 585.

Since analysis by gas chromatography of the product appeared to give an incorrect distribution of the higher molecular weight components, a small sample was treated with acetic anhydride to convert it to the corresponding 2-acetoxy derivative. The composition by gas chromatography now appeared reasonable and was as follows:

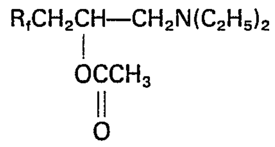

| | Area % |
|---|---|
| $R_f=C_4F_9$ | 1.3 |
| $C_6F_{13}$ | 39.4 |
| $C_8F_{17}$ | 31.8 |
| $C_{10}F_{21}$ | 15.4 |
| $C_{12}F_{25}$ | 5.6 |
| $C_{14}F_{29}$ | 1.5 |
| $C_{16}F_{33}$ | 0.4 |
| $C_{18}F_{37}$ | 0.1 |
| Unknowns | 4.5 |

The calculated average molecular weight of the perfluoroalkyl-2-hydroxypropyldiethylamines was 547.

The N,N-diethyl-3-perfluoroalkyl-2-hydroxypropylamines (176 g; 0.3 mol) were acetylated with acetic anhydride (76.5 g; 0.75 mol) by a process similar to that of Example 2, resulting in 182 g of product.

Analysis: 1.53 milliequivalents amine/g and apparent molecular weight of 653.6.

Thirty-three grams (0.05 mol) of the foregoing amines were diluted with 15 g isopropyl alcohol. Peracetic acid 40% solution (10.5 g; 0.055 mol) was added gradually to the amine solution while cooling in a cold water bath. The solution was then warmed to 50 to 53°C and held for 2 hours. The solution product was diluted with 20 g ethylene glycol and bottled. The calculated amine N-oxide content of the solution was 39%.

Example 7

N-(3-perfluoroalkyl-2-acetoxypropyl)piperidine N-oxides

The intermediate 3-perfluoroalkylpropylene oxides were prepared similarly to those described above; however, without isolation, they were reacted with piperidine (86.9 g; 1.02 mols) by heating the reaction mass at 84±2°C for 4 hours. The product was worked up by diluting the reaction mass with 1.5 liters of water and then cooling to precipitate the product as a grained solid. The product settled rapidly, was washed two times by decantation, reslurrying each time in fresh water. The product was isolated by filtration and dried in air at room temperature. The dry weight was 338 g.

Analysis: For a mixture of telomers:

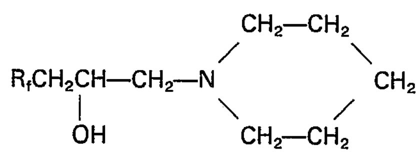

with a calculated average molecular weight of 556 the milliequivalents of amine/g:

| | |
|---|---|
| Calculated: | 1.80 |
| Found: | 1.69 |

Thus the apparent purity was 93.9% and the yield was 85.5% of theory.

The 3-perfluoroalkyl-2-hydroxypropylpiperidines (237 g; 0.4 mol) were reacted with acetic anhydride (81.6 g; 0.8 mol) by heating 2-1/2 hours at 84±3°C. The reaction mass was drowned into water and neutralized with caustic soda by the general procedure given as Example 2 to recover 253 g of an oil product.

Analysis: For a calculated average molecular weight of 598 the milliequivalents of amine/g:

| | |
|---|---|
| Calculated: | 1.67 |
| Found: | 1.55 |

Thus the yield was 94% of theory and the apparent purity was 92.8%.

To a solution mix of N-(3-perfluoroalkyl-2-acetoxypropyl)piperidines (6) g; 0.095 mol) and isopropyl alcohol (40 g) was added gradually peracetic acid 40% solution (21 g; 0.11 mol). The exothermic reaction increased the temperature of the solution mix to 55°C. The reaction was further heated at 53±3°C for 3 hours. The product was a light yellow, slightly cloudy viscous liquid. The calculated amine N-oxide content of the solution was about 47%.

Example 8

N,N-dimethyl(3-perfluoroalkyl-2-acetoxypropyl)amine N-oxides

The general procedure for preparing the subject dimethylamine derivatives from iodohydrins (500 g; 0.83 mol) followed closely Example 7 in which the intermediate propylene oxide was not isolated. The amine product was isolated initially by diluting the final reaction mass with water (1200 cc) to obtain a heavy product layer containing some isopropyl alcohol which was separated in a separatory funnel to obtain 648 g of crude product. The crude product was subjected to distillation to maximum pot temperature of 140°C at atmospheric pressure, using a slow stream of nitrogen, to strip off excess dimethyl amine and isopropyl alcohol. The final product weight was 387. The product became a soft pasty solid on standing at room temperature.

Analysis: For a mixture of Telomers:

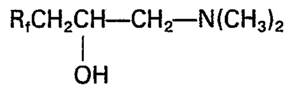

with a theoretical average molecular weight of 518 the milliequivalents of amine/g:

| | |
|---|---|
| Calculated: | 1.93 |
| Found: | 1.68 |

Thus, the apparent purity was 87% and the yield was 78.3% of theory based upon the starting iodohydrin.

The N,N-dimethyl(3-perfluoro-2-hydroxypropyl)amines (376 g; 0.63 mol) were acetylated with acetic anhydride by the procedure of Example 2. The oil product (398 g) became a soft paste on standing at room temperature. The titration for milliequivalents/gram was 1.56 indicating an apparent average molecule weight of 641 and a yield recovery of 98.6%.

A solution of N,N-dimethyl(3-perfluoroalkyl-2-acetoxypropyl)amines (32 g; 0.05 mol) and isopropyl alcohol (25 g) was treated with peracetic acid 40% solution (10.5 g; 0.055 mol) at 57±3°C for 2 hours. The calculated amine N-oxide content of the solution was 42.7%.

Example 9

N,N-bis(hydroxyethyl) (3-Perfluoroalkyl-2-hydroxypropyl)amine N-oxides

A mixture of 3-perfluoroalkylpropylene oxides (111 g. ca. 0.2 mol) was reacted with diethanolamine (42 g; 0.4 mol) by heating at 90°C for 12 hours. The initial two-phase emulsion became a homogeneous light yellow, clear, thick liquid at the end of the heating period. Excess diethanolamine was removed by extracting the reaction mass two times as 60 to 70°C with water containing approximately 10% isopropyl alcohol to aid in breaking gel formation. The crude product weight was 155.5 g.

Analysis: For N,N-bis(hydroxyethyl) (3-perfluoroalkyl-2-hydroxypropyl)amines with a calculated average molecular weight of 575 the milliequivalents of amine/g:

| | |
|---|---|
| Calculated: | 1.74 |
| Found: | 1.23 |

indicating a purity of 70.7%. The product yield, based upon the amine titration, was 95.6%.

To a solution mix of the N,N-bis(hydroxyethyl) (3-perfluoroalkyl-2-hydroxypropyl)amines (40 g) 0.049 mol) and isopropyl alcohol (40 g) was added a 30% hydrogen peroxide solution (7.0 g; and 0.062 mol). The reaction mixture was heated 6 hours at 73±3°C. The calculated concentration of amine N-oxide in the solution was 34%.

Example 10

N,N-dimethyl(3-perfluorohexyl-2-acetoxypropyl)amine N-oxide

N,N-dimethyl(3-perfluorohexyl-2-hydroxypropyl)amine (156 g; 0.35 mol) was obtained at about 95% purity by vacuum distillation and fractionation of N,N-dimethyl(3-perfluoroalkyl-2-hydroxypropyl)amines which had been prepared substantially as described in Example 1. The resulting amine was reacted with acetic anhydride (51 g; 0.5 mol) for 3 hours at 80°C. The reaction solution was drowned into 600 cc cold water and neutralized to pH 7.2 with 30% sodium hydroxide solution to cause the acetoxy derivative to separate. The product was separated in a separatory funnel, washed once with 5% brine solution and again separated. The recovered weight was 167 g.

Analysis: Titration for milliequivalents amine/g:

| | |
|---|---|
| Calculated: | 2.06 |
| Found: | 2.00 |

Thus, the indicated purity was 97.1% and the yield was 95.4% theory.

To a solution of N,N-dimethyl(3-perfluorohexyl-2-acetoxypropyl)amine (49 g; 0.103 mol) and isopropyl

alcohol (20 g) was added gradually peracetic acid 40% solution (21 g; 0.11 mol). The heat of reaction raised the temperature to 55°C. The solution mixture was then held at 60°C for 1-1/2 hours, cooled and bottled. The calculated active ingredients of the solution was 55.8%.

Example 11

N,N-diethyl(3-perfluorohexyl-2-acetoxypropyl)amine N-oxide

3-Perfluorohexylpropylene oxide (38 g; 0.1 mol) which had been obtained from pure 3-perfluorohexyl iodide via the iodohydrin and then purified by vacuum distillation was reacted with diethylamine (8.0 g; 0.11 mol) for 7 hours at 70 to 75°C and then 5 hours at 90 to 95°C. Gas chromatographic analysis still showed the presence of 2.4% of the unreacted starting propylene oxide. Without isolation, the product was acetylated with acetic anhydride (11.2 g; 0.11 mol) by heating at 80 to 85°C for 30 minutes. The solution was cooled to 60 to 65°C and then diluted with isopropyl alcohol (16 g). Hydrogen peroxide 30% solution (13.6 g; 0.12 mol) was added and the solution then heated 7 hours at 70 to 75°C. The calculated amine oxide content of the solution was 53.7%.

Example 12

N-butyl-N-methyl(3-perfluorohexyl-2-acetoxypropyl)amine N-oxide

The reaction procedure of Example 11 was repeated using N-methyl-N-butylamine instead of diethylamine.

Example 13

N,N-bis(hydroxyethyl) (3-perfluorohexyl-2-hydroxypropyl)amine N-oxide

3-Perfluorohexylpropylene oxide (38 g; 0.1 mol) was reacted with diethanolamine (11.6 g; 0.11 mol) in the presence of isopropyl alcohol (8 g) at 55 to 60°C for about 5 hours. The product was isolated by drowning into 5% brine solution and the product separated at about 50°C. A gas chromatographic analysis showed the presence of unreacted starting propylene oxide; however, the product apparently did not elute from the column and thus a quantitative determination was not made. Titration for milliequivalents of amine/g gave 1.67 while theory was 2.08 and thus showed a purity of 80.3%.

This material (30 g; 0.05 mol) was diluted with isopropyl alcohol (25 g) and oxidized with peracetic acid 40% solution (10.5 g. 0.055 mol) for 2 hours at 50°C. An amine titration for product gave an amine N-oxide content of 37.3%.

The Ross-Miles foam test on the product at 0.1% concentration in deionized water at 25°C gave 280 mm of foam which persisted for 10 minutes. Thus, the product has excellent foaming properties in water.

Example 14

N,N-dimethyl(3-perfluorooctyl-2-hydroxypropyl)amine N-oxide

The amine for this example was obtained at about 90% purity by vacuum fractional distillation of the N,N-dimethyl(3-perfluoroalkyl-2-hydroxypropyl)amines which had been prepared substantially as described in Example 1. To a solution mix of that amine (27 g; 0.05 mol) in isopropyl alcohol (25 g) at 45 to 50°C was added peracetic acid 40% solution (10.5 g; 0.055 mol). The solution was then heated 2 hours longer at 50±3°C. The calculated amine N-oxide content of this solution was 43%.

Example 15

N,N-dimethyl(3-perfluorooctyl-2-acetoxypropyl)amine N-oxide

N,N-dimethyl(3-perfluorooctyl-2-hydroxypropyl)amine corresopnding to that used in Example 14 was acetylated with acetic anhydride by the procedure of Example 2 and then vacuum distilled (75 to 80°C at 0.4—0.5 mm) to obtain material of about 94% purity as the perfluorooctyl derivative and 6% as perfluorohexyl derivative.

To a solution of N,N-dimethyl(3-perfluorooctyl-2-acetoxypropyl)amine (47.5 g; 0.08 mol) in isopropyl alcohol (20 g) was added gradually peracetic acid 40% solution (17 g) 0.09 mol) while maintaining the temperature at 50°C. Heating at 50°C was continued for 2 hours. The colorless solution was cooled and bottled. The calculated amine.N-oxide content of the solution was 54.8%. Surface tension data recorded in Table I show the product has outstanding properties.

Example 16

N-(3-perfluorooctyl-2-hydroxypropyl)piperidine N-oxide

The general procedure of Example 7 was followed, starting with 3-perfluorooctylpropylene iodohydrin. The product was isolated as a solid and air dried. Based upon titration for amine the purity was 94.4% and the yield of product was 89% of theory.

To the hydroxy derivative (89 g; 0.15 mol) was added acetic anhydride (30.8 g; 0.30 mol). After allowing the initial exothermic reaction to subside, the reaction was heated for 2 hours at 80°C. The reaction mass was drowned in cold water, neutralized with caustic soda to pH 7.5 and separated in the separatory funnel as a hot melt. The product crystallized on standing and cooling.

To a solution of N-(3-perfluorooctyl-2-acetoxypropyl)piperidine (26.3 g; 0.04 mol) and isopropyl alcohol (25 g) was added peracetic acid 40% solution (8.4 g; 0.044 mol). The reaction mass was heated 2

hours at 53±3°C to complete the oxidation. The calculated amine N-oxide content of the solution was 45.1%.

Example 17
N,N'-bis(3-perfluorohexyl-2-acetoxypropyl)-N,N'-dimethylhexamethylenediamine-N,N'-dioxide

In accordance with the general reaction procedure of Example 11, 3-perfluorohexylpropylene oxide (38 g; 0.1 mol) was reacted for 7-1/2 hours at 95±3°C with N,N'-dimethylhexamethylenediamine (7.5 g; 0.05 mol). This reaction was followed by acetylation with acetic anhydride (0.095 mol), dilution with isopropyl alcohol and reaction with hydrogen peroxide 30% solution (12.5 g; 0.11 mol).

Example 18
N,N'-bis(3-perfluorohexyl-2-acetoxypropyl)-N,N'-diethylhexamethylenediamine-N,N'-dioxide

Following the general procedure of Example 11, 3-perfluorohexylpropylene oxide (38 g; 0.1 mol) was reacted 9 hours at 95±3°C with N,N'-diethylhexamethylenediamine (9.0 g; 0.05 mol). After acetylation the oxidation was carried out using peracetic acid 40% solution (21 g; 0.11 mol) at 50°C for 2 hours.

TABLE I
Surface tension of amine oxide fluorosurfactants

| | | Surface tension (dynes/cm) at concentrations (%) | | |
|---|---|---|---|---|
| Example | Amine oxide | 0.1 | 0.01 | 0.001 |
| 1. | N,N-Dimethyl(3-Perfluoroalkyl-2-Hydroxypropyl)Amine N-Oxides | 17.3 | 18.4 | 36.1 |
| 2. | N,N-Dimethyl(3-Perfluoro-2-Acetoxypropyl)Amine N-Oxides | 17.0 | 18.8 | 25.3 |
| 3. | N,N-Dimethyl(3-Perfluoroalkyl-2-Acetoxypropyl)Amine N-Oxides | 16.6 | 17.3 | 21.5 |
| 4. | N-Butyl-N-Methyl(3-Perfluoroalkyl-2-Acetoxypropyl)Amine N-Oxides | 17.2 | 17.5 | 23.9 |
| 5. | N-(3-Perfluoroalkyl-2-Acetoxypropyl)Piperidine N-Oxides | 17.1 | 21.0 | 31.7 |
| 6. | N,N-Diethyl(3-Perfluoroalkyl-2-Acetoxypropyl)Amine N-Oxides | 17.8 | 18.5 | 21.6 |
| 7. | N-(3-Perfluoroalkyl-2-Acetoxypropyl)Piperidine N-Oxides | 17.8 | 18.5 | 20.7 |
| 8. | N,N-Dimethyl(3-Perfluoroalkyl-2-Acetoxypropyl)Amine N-Oxides | 18.1 | 19.3 | 26.0 |
| 9. | N,N-Bis(Hydroxyethyl) (3-Perfluoroalkyl-2-Hydroxypropyl)-Amine N-Oxides | 16.8 | 17.5 | 24.9 |
| 10. | N,N-Dimethyl(3-Perfluorohexyl-2-Acetoxypropyl)Amine N-Oxide | 16.4 | 18.1 | 44.6 |
| 11. | N,N-Diethyl(3-Perfluorohexyl-2-Acetoxypropyl)Amine N-Oxide | 16.4 | 20.7 | 45.6 |
| 12. | N-Butyl-N-Methyl(3-Perfluorohexyl-2-Acetoxypropyl)Amine N-Oxide | 16.9 | 21.6 | 41.7 |

TABLE I (cont.)
Surface tension of amine oxide fluorosurfactants

| Example | Amine oxide | Surface tension (dynes/cm) at concentrations (%) 0.1 | 0.01 | 0.001 |
|---|---|---|---|---|
| 13. | N,N-Bis(Hydroxyethyl) (3-Perfluorohexyl-2-Hydroxypropyl)-Amine N-Oxide | 15.7 | 29.7 | 57.2 |
| 14. | N,N-Dimethyl(3-Perfluorooctyl-2-Hydroxypropyl)Amine N-Oxide | 16.6 | 17.5 | 21.1 |
| 15. | N,N-Dimethyl(3-Perfluorooctyl-2-Acetoxypropyl)Amine N-Oxide | 16.2 | 16.4 | 17.5 |
| 16. | N-(3-Perfluorooctyl-2-Hydroxypropyl)Piperidine N-Oxide | 18.0 | 18.2 | 22.0 |
| 17. | N,N'-Bis(3-Perfluorohexyl-2-Acetoxypropyl)-N,N'-Dimethylhexamethylenediamine-N,N'-Dioxide | 17.0 | 18.4 | 21.1 |
| 18. | N,N'-Bis(3-Perfluorohexyl-2-Acetoxypropyl)-N,N'-Diethylhexamethylenediamine-N,N'-Dioxide | 17.5 | 17.5 | 25.3 |

## Claims

1. A 1,1,2,3,3-pentahydroperfluoroalkylamine N-oxide represented by the formula:

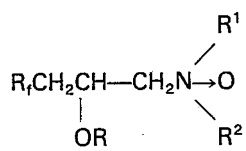

wherein

$R_f$ is $C_3$—$C_{20}$ perfluoroalkyl;

R is H or alkanoyl containing 1—4 carbons;

$R^1$ and $R^2$ are the same or different $C_1$—$C_6$ alkyl or $C_1$—$C_6$ hydroxyalkyl, or together with the N atom to which they are bonded form a piperidine, morpholine or N-alkyl($C_1$—$C_4$) piperazine ring; or

$R^1$ is $C_1$—$C_6$ alkyl or $C_1$—$C_6$ hydroxyalkyl and

$R^2$ is 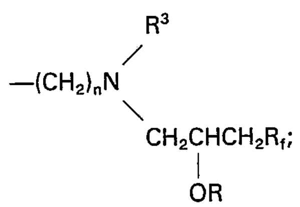

$R^3$ is $C_1$—$C_6$ alkyl or $C_1$—$C_6$ hydroxyalkyl; and

n is an integer from 1—8; and mixtures thereof.

2. Mixtures of amine N-oxides as claimed in claim 1 wherein 80—90% of the $R_f$ groups contain 6—12 carbons.

3. Mixtures of amine N-oxides as claimed in claim 2 wherein said $R_f$ groups contain 6—10 carbon atoms.

4. Mixtures of amine N-oxides as claimed in claim 1, 2 or 3 wherein the average of the chain lengths in the $R_f$ groups is 6—8 carbons.

5. An amine N-oxide or mixtures of amine N-oxides as claimed in any one of the preceding claims wherein R is acetyl.

6. A process for reducing surface tension in an aqueous system comprising adding thereto an effective amount of an amine N-oxide or mixture of amine N-oxides as claimed in any of the preceding claims.

7. A process for generating foam in an aqueous system comprising adding thereto an effective amount of an amine N-oxide or mixture or amine N-oxides is claimed in any one of claims 1 to 5 and agitating the same.

8. A process for preparing an amine N-oxide or mixtures of amine N-oxides as defined in any of claims 1 to 5, which comprises reacting an oxidizing agent with an amine or mixture of amines represented by the formula:

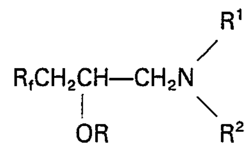

wherein $R_f$, R, $R_1$ and $R_2$ are as defined in claim 1.

9. A process according to claim 8, wherein said oxidizing agent is peracetic acid.

10. A process according to claim 8, wherein said oxidizing agent is hydrogen peroxide.

**Patentansprüche**

1. 1,1,2,3,3-Pentahydroperfluoralkylamin-N-oxid, wiedergegeben durch die Formel

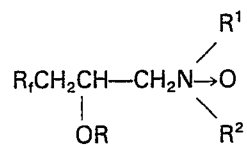

worin

$R_f$ $C_3$—$C_{20}$-Perfluoralkyl ist;

R H oder Alkanoyl mit 1—4 Kohlenstoffen ist;

$R^1$ und $R^2$ gleiches oder verschiedenes $C_1$—$C_6$-Alkyl oder $C_1$—$C_6$-Hydroxyalkyl sind oder zusammen mit dem N-Atom, an welches als gebunden sind, einen Piperidin-, Morpholin- oder N-Alkyl($C_1$—$C_4$)piperazinring bilden; oder

$R^1$ $C_1$—$C_6$-Alkyl oder $C_1$—$C_6$-Hydroxyalkyl ist und

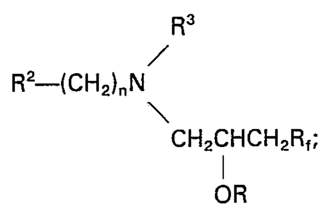

ist;

$R^3$ $C_1$—$C_6$-Alkyl oder $C_1$—$C_6$-Hydroxyalkyl ist; und

n eine ganze Zahl von 1 bis 8 ist;

und Mischungen davon.

2. Mischungen von Amin-N-oxiden, wie in Anspruch 1 beansprucht, worin 80 bis 90% der $R_f$-Gruppen 6—12 Kohlenstoffe enthalten.

3. Mischungen von Amin-N-oxiden, wie in Anspruch 2 beansprucht, worin die $R_f$-Gruppen 6—10 Kohlenstoffatome enthalten.

4. Mischungen von Amin-N-oxiden, wie in Anspruch 1, 2 oder 3 beansprucht, worin der Durchschnitt der Kettenlängen der $R_f$-Gruppen 6—8 Kohlenstoffe ist.

5. Amin-N-oxid oder Mischungen von Amin-N-oxiden, wie in einem der vorstehenden Ansprüche beansprucht, worin R Acetyl ist.

6. Verfahren zur Verminderung der Oberflächenspannung in einem wässrigen System durch Hinzufügen einer wirksamen Menge eines Amin-N-oxids oder von Mischungen von Amin-N-oxiden, wie in einem der vorstehenden Ansprüche beansprucht.

7. Verfahren zur Bildung von Schaum in einem wässrigen System durch Hinzufügen einer wirksamen

Menge eines Amin-N-oxids oder von Mischungen von Amin-N-oxiden, wie in einem der Ansprüche 1 bis 5 beansprucht, und Rühren desselben.

8. Verfahren zur Herstellung eines Amin-N-oxids oder von Mischungen von Amin-N-oxiden, wie in einem der Ansprüche 1 bis 5 beansprucht, durch Reagieren eines Oxidationsmittels mit einem Amin oder einer Mischung von Aminen, wiedergegeben durch die Formel

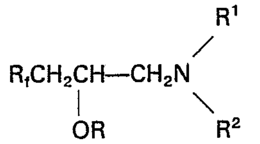

worin $R_f$, R, $R_1$ und $R_2$ wie in Anspruch 1 definiert sind.

9. Verfahren nach Anspruch 8, worin das Oxidationsmittel Peressigsäure ist.

10. Verfahren nach Anspruch 8, worin das Oxidationsmittel Wasserstoffperoxid ist.

**Revendications**

1. Un N-oxyde de 1,1,2,3,3-pentahydroperfluoroalkylamine représenté par la formule:

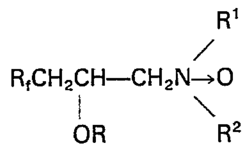

dans laquelle

$R_f$ est un groupe perfluoroalkyle en $C_3$—$C_{20}$;

R est H ou un groupe alcanoyle contenant 1—4 atomes de carbone;

$R^1$ et $R^2$, identiques ou différente, sont chacun un groupe alkyle en $C_1$—$C_6$ ou hydroxyalkyle en $C_1$—$C_6$, ou, avec l'atome d'azote auquel ile sont reliés, formant un noyau de pipéridine, de morpholine ou de N-(alkyl en $C_1$—$C_4$)-pipérazine; ou bien

$R^1$ est un groupe alkyle en $C_1$—$C_6$ ou hydroxyalkyle en $C_1$—$C_6$

et $R^2$ est 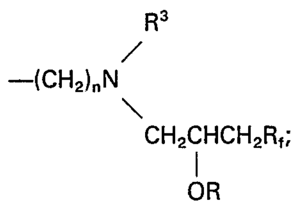

$R^3$ est un groupe alkyle en $C_1$—$C_6$ ou hydroxyalkyle en $C_1$—$C_6$; et

n est ou nombre entier égal à 1—8;

et leurs mélanges.

2. Mélanges de N-oxydes d'amines tels que revendiqués dans la revendication 1, dans lesquels 80—90% des groupes $R_f$ contiennent 6—12 atomes de carbone.

3. Mélanges de N-oxydes d'amines tels que revendiqués dans la revendication 2 dans lesquels lesdits groupes $R_f$ contiennent 6—10 atomes de carbone.

4. Mélanges de N-oxydes d'amines tels que revendiqués dans la revendication 1, 2 ou 3, dans lesquels la moyenne les longueurs de chaîne des groupes $R_f$ est de 6 à 8 atomes de carbone.

5. Un N-oxyde d'amine ou des mélanges de N-oxydes d'amines tels que revendiqués dans l'une quelconque des revendications précédentes dans lesquels R est le groupe acétyle.

6. Un procédé pour réduire la tension superficielle dans un système aqueux consistant à ajouter à ce système une quantité efficace d'un N-oxyde d'amine ou d'un mélange de N-oxydes d'amines tel que revendiqué dans l'une quelconque des revendications précédentes.

7. Un procédé pour produire de la mousse dans un système aqueux, consistant à ajouter à ce système une quantité efficace d'un N-oxyde d'amine ou d'un mélange de N-oxydes d'amines tel que revendiqué dans l'une quelconque des revendications 1 à 5, et à l'agiter.

8. Un procédé pour préparer un N-oxyde d'amine ou des mélanges de N-oxydes d'amines tels que définis dans l'une quelconque des revendications 1 à 5, qui consiste à faire réagir un agent oxydant avec une amine ou un mélange d'amines représentées par la formule:

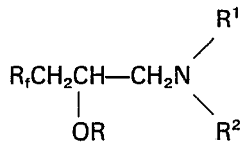

dans laquelle $R_f$, R, $R_1$ et $R_2$ sont tels que définis dans la revendication 1.

9. Un procédé selon la revendication 8, dans lequel ledit agent oxydant est l'acide peracétique.

10. Un procédé selon la revendication 8, dans lequel ledit agent oxydant est le peroxyde d'hydrogène.